Europäisches Patentamt

European Patent Office

Office européen des brevets

(19)

(11) Veröffentlichungsnummer: **0 267 518**
**B1**

(12) **EUROPÄISCHE PATENTSCHRIFT**

(45) Veröffentlichungstag der Patentschrift: **16.01.91**

(51) Int. Cl.⁵: **C 07 C 303/30, C 07 C 305/02**

(21) Anmeldenummer: **87116132.9**

(22) Anmeldetag: **03.11.87**

(54) **Verfahren zur Sulfatierung von Teilestern aliphatischer, mehrwertiger Alkohole.**

(30) Priorität: **13.11.86 DE 3638742**
**17.07.87 DE 3723702**

(43) Veröffentlichungstag der Anmeldung:
**18.05.88 Patentblatt 88/20**

(45) Bekanntmachung des Hinweises auf die
Patenterteilung:
**16.01.91 Patentblatt 91/03**

(84) Benannte Vertragsstaaten:
**AT BE CH DE ES FR GB IT LI NL SE**

(56) Entgegenhaltungen:
**DE-C- 689 511**
**US-A-3 595 903**
**US-A-3 763 208**

(73) Patentinhaber: **HOECHST**
**AKTIENGESELLSCHAFT**
**Postfach 80 03 20**
**D-6230 Frankfurt am Main 80 (DE)**

(72) Erfinder: **Stühler, Herbert, Dr.**
**Hochfellnstrasse 12**
**D-8269 Burgkirchen (DE)**
Erfinder: **Dullinger, Klaus, Dr.**
**Bahnhofstrasse 18**
**D-8265 Neuötting (DE)**

Courier Press, Leamington Spa, England.

## Beschreibung

Die Erfindung betrifft ein Verfahren zur Sulfatierung von Teilestern aliphatischer, mehrwertiger Alkohole.

Sulfatierte Teilester von mehrwertigen, aliphatischen Alkoholen, beispielsweise Fettsäuremonoglycerinester-Sulfate, sind wertvolle Tenside, die als Waschrohstoffe, Seifen oder Flotationsmittel verwendet werden. Zu ihrer Herstellung sind verschiedene Verfahren bekannt:

Nach US—PS 2,242,979 wird ein Sulfoniermittel, beispielsweise Schwefelsäure, mit einem mehrwertigen Alkohol bei 30°C gemischt und anschließend mit Fettsäure oder Fetten bzw. Ölen unter Erwärmung reagieren gelassen, danach neutralisiert. Der Überschuß an Schwefelsäure soll so groß sein, daß am Ende der Reaktion immer noch eine 99,3 gew.-%ige Säure vorliegt.

Nach DE—PS 689 511 wird ein Gemisch von gesättigten Fettsäuren oder nach DE—PS 702 598 von Fettsäuretriglyceriden und mehrwertigen Alkoholen in einem Mischungsverhältnis, das die Bildung von freie Hydroxygruppen im Alkoholrest enthaltenden Fettsäureestern erlaubt, mit einem großen Überschuß an konzentrierter bis wasserfreier Schwefelsäure sulfoniert und anschließend mit Alkalilauge oder Stickstoffbase neutralisiert.

Nach CH—PS 273 375 werden molare Mengen Glycerin, Cocosöl und Talg in Gegenwart eines großen Überschusses an rauchender (beispielsweise ca. 20% SO₃ enthaltender) Schwefelsäure umgesetzt, auf Eis gegossen und mit wäßriger Natronlauge neutralisiert.

Nach US—PS 2,868,812 werden 1 mol eines in wesentlichen gesättigten Fett-triglycerids, 2 mol Glycerintrischwefelsäureester und 4,0 bis 4,8 mol Schwefelsäure-Monohydrat bei 30 bis 65°C umgesetzt, dann mit Natronlauge neutralisiert.

Nach US—PS 2,634,287 wird beispielsweise 1 mol Glycerinmonolaurat in flüssigem SO₂ bei wenigstens 20°C mit 1 mol SO₃ in flüssigem SO₂ umgesetzt, das SO₂ abgedampft und das Reaktionsprodukt mit Natronlauge neutralisiert.

Nach GB—PS 364 107, Beispiel 3, wird Glycerinmonolaurat mit einem Überschuß an konzentrierter Schwefelsäure bei 60°C umgesetzt, auf Eis gegossen und mit Butylacetat extrahiert. Dieser Extrakt wird mit wäßriger Natriumcarbonatlösung neutralisiert und vom Butylacetat abgetrennt.

Nach K. Engel und W. Ruback; "Fette, Seifen, Anstrichmittel" 88 (1986), Seite 21/22 werden Fettsäuremonoglycolester mit Chlorsulfonsäure umgesetzt, wobei sich Temperaturen von −20 bis 0°C als günstig erwiesen. Nach Neutralisation werden 30 %ige wäßrige Natriumsalzlösungen der Fettsäureglycolestersulfate erhalten.

Schließlich wird nach A. K. Biswas und B. K. Nukherji; The Journal of the American Oil Chemists Society 37 (1960), Seite 172, rechte Spalte, ein erheblicher Überschuß Pyridin-Schwefeltrioxid-Komplex bei 10°C zu einem ungesättigten Fettsäuremonoglycerid gegeben, das Reaktionsgemisch in heißem n-Butanol gelöst und mit heißer Natriumhydroxidlösung neutralisiert.

Die Verfahren nach dem Stand der Technik haben mindestens einen der folgenden Nachteile:

Bildung erheblicher Mengen Natriumsulfats, die das Abwasser belasten.

Bildung von HCl, deren Entsorgung Kosten verursacht.

Arbeiten unter Druck mit aggressiven Medien erforderlich.

Die Aufarbeitung zur trockenen Substanz ist energieaufwendig, da erhebliche Mengen Wassers verdampft werden müssen.

Es fällt ein Gemisch organischer Lösungsmittel an, das vor der Wiederverwendung destillativ getrennt werden muß.

Es ist ein besonderer Reaktionsschritt zur Gewinnung des Sulfierungsmittels erforderlich.

Es wurde nun ein Verfahren gefunden, das die oben beschriebenen Nachteile nicht aufweist und mit guten Ausbeuten hellfarbige Produkte ergibt. Das neue Verfahren zur Sulfatierung primärer, freier OH-Gruppen von Teilestern aliphatischer, mehrwertiger Alkohole mit SO₃ in Gegenwart eines Lösungsmittels ist dadurch gekennzeichnet, daß als Lösungsmittel mindestens eine Verbindung eingesetzt wird, die bei 98 kPa Druck einen Siedepunkt von 40 bis 200°C aufweist, unter den gewählten Reaktionsbedingungen flüssig ist und neben mindestens einem dreiwertigen N-Atom, das ausschließlich mit C-Atomen verbunden ist, nur C-, H- und gegebenenfalls Ethersauerstoff-Atome enthält.

Als aliphatische, mehrwertige Alkohole mit mehreren primären, freien OH-Gruppen kommen beispielsweise in Frage Ethylenglykol, Glycerin, Diglycerin, durch Wasserabspaltung aus Glycerin entstandene Polyglycerine, Pentaerythrit und Zukkeralkohole wie Mannit oder Sorbit. Es können auch die Ethylenoxid-Umsetzungsprodukte dieser Alkohole eingesetzt werden. Vorzugsweise wird als Teilester mindestens ein Carbonsäuremonoester des Glycerins, Diglycerins, Ethylenglykols oder Diethylenglykols verwendet.

Als Veresterungskomponente der aliphatischen, mehrwertigen Alkohole sind aliphatische Carbonsäuren mit 1 bis 25 C-Atomen oder arylaliphatische Carbonsäuren mit 7 bis 16 C-Atomen geeignet, besonders Monocarbonsäuren mit endständigen COOH-Gruppen wie zum Beispiel Essigsäure, Butan-, Octan-, Laurin-, Palmitin-, Stearinsäure. Diese Säuren können in der C-Kette Doppelbindungen enthalten, wie beispielsweise Öl-, Linol-, Linolen-, Brassidin-, oder Erucasäure. Die aliphatischen Carbonsäuren können sekundäre Hydroxylgruppen enthalten, wie beispielsweise Hydroxystearinsäure oder Ricinolsäure. Bevorzugt werden Teilester aliphatischer, mehrwertiger Alkohole mit aliphatischen Monocarbonsäuren, die 8 bis 22 C-Atome enthalten und Doppelbindungen beziehungsweise sekundäre Hydroxylgruppen enthalten können, eingesetzt. Es können auch Gemische solcher Teilester verwendet werden, sofern diese noch freie primäre Hydroxylgruppen enthalten, beispielsweise ein Gemisch von Glycerinomonocarbonsäureester und Glycerindicarbonsäureester.

Die Umsetzung mit $SO_3$ findet erfindungsgemäß in Gegenwart eines Lösungsmittels statt, das bei 98 kPa Druck einen Siedepunkt von 40 bis 200°C aufweist, unter den gewählten Reaktionsbedingungen flüssig ist und neben mindestens einem dreiwertigen N-Atom, das ausschließlich mit C-Atomen verbunden ist, nur C-, H- und gegebenenfalls Ethersauerstoff-Atome enthält. Beispiele für geeignete Verbindungen sind: Triethylamin; Di-isopropyl-ethyl-amin; Tri-isopropylamin; Tri-n-propyl-amin; Tri-isobutyl-amin; Methyl-diethyl-amin; N,N-Dimethyl-cyclohexylamin; N,N-Dimethylanilin; Pyrimidin; Pyridin; N-Methyl-piperidin; N-Methyl-pyrrol; 3-Methylpyrazin; 1-Methylimidazol; N-Methylpyrrolidin; N-Methyl- und N-Ethyl-morpholin. Vorzugsweise werden Verbindungen verwendet, bei denen ein Stickstoffatom mit 3 Kohlenstoffatomen verbunden ist.

Unter den gewählten Reaktionsbedingungen sollen die als Lösungsmittel eingesetzten stickstoffhaltigen Verbindungen flüssig sein. Da die Umsetzung des Teilesters mit dem $SO_3$ vorteilhaft bei 0 bis 60°C erfolgt, können stickstoffhaltige Verbindungen als Lösungsmittel eingesetzt werden, deren Siedepunkt bei 98 kPa bei 10°C oder darüber liegt. Nach oben ist der Siedepunkt bei dem genannten Druck durch wirtschaftliche Erwägungen begrenzt und dadurch, daß Verbindungen mit einem hohen Siedepunkt in der Regel unter den gewählten Reaktionsbedingungen nicht mehr flüssig sind. Vorteilhaft wird ein stickstoffhaltiges Lösungsmittel eingesetzt, das bei 98 kPa Druck einen Siedepunkt von 40 bis 200°C, insbesondere von 60 bis 150°C aufweist. Das $SO_3$ kann fest oder flüssig verwendet werden, vorteilhaft wird es gasförmig, verdünnt mit einem Inertgas, beispielsweise Stickstoff, Luft oder Argon, eingesetzt. Gute Ergebnisse werden erhalten, wenn die Gasmischung 1 bis 30 Vol.-% und insbesondere 2 bis 10 Vol.-% $SO_3$ enthält. Eine solche Gasmischung kann beispielsweise erzeugt werden, indem ein Stickstoff-Strom über gegebenenfalls erwärmtes $SO_3$ oder Oleum geleitet wird. Das Inkontaktbringen der im stickstoffhaltigen Lösungsmittel gelösten Teilester aliphatischer, mehrwertiger Alkohole mit dem $SO_3$-haltigen Gasstrom kann auf verschiedenen Wegen erfolgen, beispielsweise durch Einleiten oder Überleiten über die Flüssigkeit, die zweckmäßigerweise durch Rühren bewegt wird, oder durch Herabrieselnlassen eines Flüssigkeitsfilms, der mit dem $SO_3$-haltigen Gasgemisch entweder im Gleich- oder Gegenstrom geführt wird.

Je 1 mol primäre freie OH-Gruppe des Teilesters des aliphatischen, mehrwertigen Alkohols werden vorteilhaft 0,9 bis 1,2 mol $SO_3$ verwendet. Unter 0,9 mol $SO_3$ werden im allgemeinen unerwünscht niedrig sulfatierte Produkte erhalten, oberhalb 1,2 mol $SO_3$ treten häufig unerwünschte weitere Reaktionen ein, auch wird nach der Neutralisation die Salzbelastung des Verfahren unnötig hoch.

Die Temperatur während der Umsetzung des Teilesters des aliphatischen, mehrwertigen Alkohols mit dem $SO_3$ kann in weiten Grenzen schwanken. Vorteilhaft erfolgt sie bei 0 bis 60°C, insbesondere bei 10 bis 40°C. Zu hohe Temperaturen begünstigen unerwünschte Nebenreaktionen, zu niedrige Temperaturen erhöhen unnötig die Reaktionsdauer. Die erfindungsgemäß Umsetzung kann unter verschiedenem Druck erfolgen, wegen des geringen apparativen Aufwandes wird im allgemeinen der normale Atmosphärendruck bevorzugt.

Das Fortschreiten der Sulfatierung wird zweckmäßig durch analytische Methoden verfolgt, beispielsweise durch Titration einer Probe, die vorher durch Abdampfen vom Lösungsmittel befreit wurde, mit Alkalilauge. Wenn die gewünschte Menge $SO_3$ von der Reaktionsmischung aufgenommen worden ist, wird die $SO_3$-Zufuhr beendet, es kann sich dann noch eine Nachreaktionszeit von etwa 0,5 bis 3 h anschließen, während der das Reaktionsgemisch unter Einhaltung der Reaktionstemperatur oder Steigerung der Temperatur auf bis zu 120°C gegebenenfalls unter Druck weiter bewegt wird. Die Reaktionsdauer hängt von der gewählten Temperatur, der Konzentration des $SO_3$, der Art des Teilesters des aliphatischen, mehrwertigen Alkohols und der Art des Inkontaktbringens der Reaktionspartner ab. Im allgemeinen sind Zeiten von 0,5 bis 20 h, vorzugsweise 3 bis 10 h, ausreichend.

Nach Beendigung der $SO_3$-Zugabe und gegebenenfalls einer Nachreaktionszeit kann das Produkt vom Lösungsmittel, beispielsweise durch Abdestillieren, getrennt werden, es resultiert dann das Aminsalz des sulfatierten Teilesters des aliphatischen, mehrwertigen Alkohols, das als solches bereits Verwendung finden kann. In vielen Fällen ist jedoch das Alkali- oder Erdalkalisalz des sulfatierten Teilesters erwünscht. Dies wird vorteilhaft dadurch hergestellt, daß nach Beendigung der $SO_3$-Zugabe und gegebenenfalls einer Nachreaktionszeit die Reaktionsmischung mit einem Alkali- oder Erdalkalihydroxid versetzt und anschließend das Lösungsmittel abdestilliert wird. Wegen der leichten Beschaffbarkeit ist Kalium- oder Calciumhydroxid und insbesondere Natriumhydroxid bevorzugt. Gute Ergebnisse werden erhalten, wenn je 1 mol eingesetztes $SO_3$ 1 bis 1,4 Äquivalente des Alkali- oder Erdalkalihydroxids verwendet werden. Während der Abtrennung des stickstoffhaltigen Lösungsmittels, zweckmäßig durch Destillation, wird vorteilhaft der Druck so eingestellt, daß die Sumpftemperatur 30 bis 90°C beträgt. Bei höheren Temperaturen nimmt die Gefahr der Bildung von Verunreinigungen zu, vorzugsweise wird im Bereich einer Sumpftemperatur von 40 bis 70°C gearbeitet.

Das vom Reaktionsprodukt abgetrennte stickstoffhaltige Lösemittel wird nach Entfernen von Wasser mit Vorteil erneut für die erfindungsgemäße Reaktion eingesetzt. Im allgemeinen wird soviel Lösungsmittel verwendet, daß mindestens ein Drittel, vorzugsweise der gesamte Teilester des aliphatischen, mehrwertigen Alkohols bei der gewählten Reaktionstemperatur in Lösung geht. Um die Einführung größerer Wassermengen in den Prozeß zu vermeiden, kann das Alkalihydroxid im stickstoffhaltigen Lösungsmittel gelöst beziehungsweise mit diesem verdünnt zur Neutralisation benutzt werden. Anstelle des Alkalioder Erdalkalihydroxids kann auch ein quaternäres Ammoniumhydroxid Verwendung finden.

Nach der Behandlung mit Alkali-, Erdalkalioder quaternärem Ammoniumhydroxid und Abtrennen des stickstoffhaltigen Lösungsmittels, beispielsweise durch Destillation, verbleibt als Rückstand das Alkali-, Erdalkali- oder quaternäre Ammoniumsalz des sulfatierten Teilesters vom aliphatischen, mehrwertigen Alkohol als hellfarbige bis weiße Substanz von für die meisten Anwendungszwecke ausreichender Reinheit. Falls erforderlich, können sich weitere an sich bekannte Reinigungsoperationen anschließen.

Das erfindungsgemäße Verfahren ermöglicht es, hellfarbige Substanzen mit guten Tensideigenschaften, die vergleichsweise wenig Fremdsalze enthalten, herzustellen. Es kann weitgehend unter Normaldruck gearbeitet werden, lediglich die Abdestillation des Lösungsmittels kann bisweilen verminderten Druck erfordern.

Wie bereits eingangs erwähnt, eignen sich die nach dem neuen Verfahren hergestellten Substanzen beispielsweise als Waschrohstoffe, für synthetische Seifen oder als Flotationshilfsmittel.

Nachfolgende Beispiele sollen die Erfindung näher erläutern:

Beispiel 1

In einem 1 dm³ fassenden Gefäß, das mit Thermometer, Rührer Kühler und Gaseinleitungsrohr versehen ist, werden 50 g eines Produktes, das 91 Gew.-% Glycerinmonolaurat neben höher veresterten Produkten enthält (das sind 0,165 mol Glycerinmonolaurat), bei 22°C in 300 cm³ Triethylamin unter Rühren gelöst. Nun werden unter Fortsetzung des Rührens durch das Gaseinleitungsrohr, dessen Öffnung oberhalb des Flussigkeitsoberfläche endet, innerhalb 8 h eine Gasmischung, die 2,3 Vol.-% SO₃ Stickstoff enthält, eingeleitet. Aufgenommen werden 13,5 g SO₃=0,168 mol, je 1 mol Teilester werden demnach 1,02 mol SO₃ angewendet. Die Temperatur steigt auf 25°C an und wird auf dieser Höhe gehalten. Während der Reaktion werden kleine Proben entnommen, das Lösungsmittel abgedampft und im Rückstand durch Titration mit wäßriger Natronlauge die Säurezahl bestimmt. Die Einleitung des SO₃-haltigen Gasgemisches wird bei Erreichen einer Säurezahl von 145 mgKOH/g Substanz beendet, anschließend wird noch 1 h ohne Gaseinleitung weiter gerührt. Eine Probe des Reaktionsgemische enthält nach Abdampfung des Lösungsmittels 90 Gew.-% des Triethylaminsalzes vom sulfatierten Glycerinmonolaurat, bestimmt durch Zweiphasentitration nach Epton, außerdem 2,1 Gew.-% H₂SO₄, bestimmt als Sulfat. Das Reaktionsgemisch wird nun bei 22°C unter Rühren langsam tropfenweise mit 28,3 g einer 30 gew.-%igen Natronlauge in Wasser, die mit 50 cm³ Triethylamin verdünnt wurde, versetzt. Anschließend wird das Triethylamin bei 60°C Sumpftemperatur und vermindertem Druck bis zu einem Endwert von 2 kPa abdestilliert. Als Rückstand wird das Natriumsalz des sulfatierten Glycerinmonolaurats als weißes, geruchloses Pulver erhalten. Es enthält nach Analyse 90 Gew.-% Natriumsalz des sulfatierten Produktes und 3,4 Gew.-% Natriumsulfat.

Beispiel 2

Es wird verfahren wie in Beispiel 1 beschrieben, jedoch werden in das Reaktionsgefäß 70 g eines Produktes, das 90 Gew.-% Glycerinmonosterat (0,175 mol) enthält, in 235 g Triethylamin gelöst. Nun werden bis zum Erreichen einer Säurezahl von 120 mgKOH/g Substanz bei 26°C ein Gasgemisch, das 2,3 Vol.-% SO₃ neben Stickstoff enthält, unter Rühren der Flüssigkeit während mehrerer Stunden in das Reaktionsgefäß eingeleitet, anschließend noch 40 min nachgerührt. Insgesamt werden 14,3 g SO₃=0,1785 mol eingeleitet, das sind je mol Glycerinmonostearat 1,02 mol SO₃. Das Reaktionsgemisch enthält nach Abdampfung des Lösungsmittels 90 Gew.-% des Triethylaminsalzes vom sulfatierten Glycerinmonostearat, bestimmt durch Zweiphasentitration nach Epton, neben, 2,3 Gew.-% Schwefelsäure, bestimmt als Sulfat. Nu werden, wie in Beispiel 1 beschrieben, bei 22°C langsam unter Rühren 32 g einer 30 gew.-%igen Natronlauge in Wasser, die mit 50 cm³ Triethylamin verdünnt wurde, eingetropft. Das sind je mol SO₃ 1,34 Äquivalente NaOH. Anschließend wird das Triethylamin bei 60° Sumpftemperatur und vermindertem Druck bis zu 2 kPa abdestilliert. Es wird das Natriumsalz des sulfatierten Glycerinmonostearats als weißes, geruchloses Pulver erhalten, dieses enthält nach Analyse 90 Gew.-% Natriumsalz des sulfatierten Produktes, neben 3,6% Natriumsulfat.

Beispiel 3

Es wird wiederum gearbeitet wie in Beispiel 1 beschrieben. 100 g eines Produktes, das 75 Gew.-% Glycerinmonolaurat (Rest höherveresterte Produkte) enthalt, das sind 0,273 mol, werden in 242 g Triethylamin unter Rühren bei 22°C gelöst, danach bei 25°C mit einer Gasmischung, die 2,3 Vol.-% SO₃ neben Stickstoff enthält, während mehrerer Stunden begast, bis in der Reaktionsmischung eine Säurezahl von 130 mgKOH/g Substanz festgestellt wird. Insgesamt werden 23,3 g das sind 0,291 mol SO₃ eingegast, das sind je mol Teilester 1,06 mol SO₃. Anschließend wird noch 60 min ohne Gaseinleitung weitergerührt. Das Reaktionsgemisch enthält nun 75 Gew.-% des Triethylaminsalzes vom sulfatierten Glycerinmonolaurat, bestimmt durch Zweiphasentitration nach Epton, neben 2,5 Gew.-% Schwefelsäure, bestimmt als Sulfat. Analog Beispiel 1 wird dieses Reaktionsgemisch bei 22°C unter Rühren langsam tropfenweise mit 47 g einer 30 gew.-%igen Natronlauge in Wasser, die mit 50 ml Triethylamin verdünnt wurde, versetzt. Das sind 0,352 mol NaOH, oder je mol SO₃ 1,21 mol NaOH. Nach Beendigung der Neutralisation wird das Triethylamin bei 60° Sumpftemperatur und vermindertem Druck bis zu 2 kPa abdestilliert. Wiederum wird das Natriumsalz des Sulfatierten Glycerinmonolaurats als weißes, geruchloses Pulver erhalten. Der Rückstand enthält nach Analyse 70 Gew.-% des Natriumsalzes vom sulfatierten Produkt, neben 4,3 Gew.-% Natriumsulfat.

Beispiel 4

Es wird verfahren wie im Beispiel 1 beschrieben, jedoch werden in dem Reaktionsgefäß 30 g eines Produktes, das 91 Gew.-% Glycerinmonolaurat neben höher veresterten Produkten enthält (das sind 0,0993 mol Glycerinmonolaurat) bei 21°C in 250 cm³ N-Methylmorpholin unter Rühren gelöst. Dann wird innerhalb 3,5 h eine Gasmischung, die neben Stickstoff 6 Vol.-% SO₃ enthält, unter Fortsetzung des Rührens über die Oberfläche der Lösung geleitet, wobei deren Temperatur auf 21°C gehalten wird. Es werden 8,6 g SO₃ (=0,1074 mol) von der Lösung aufgenommen, das sind 1,08 mol SO₃ je 1 mol des Glycerin-Teilesters. Nach Beendigung der SO₃-Einleitung wird die Reaktionsmischung unter Rühren 1 h auf 40°C erwärmt. Eine Probe des Reaktionsgemisches enthält nach Abdampfung des Lösungsmittels 83 Gew.-% des N-Methylmorpholin-salzes vom sulfatierten Glycerin monolaurat (bestimmt durch Zweiphasentitration nach Epton) und hat eine Säurezahl von 212 mg KOH/g. Das Reaktionsgemisch wird nun mit 15,7 g einer 30 gew.-%igen wäßrigen Natronlauge, die mit 30 cm³ N-Methylmorpholin verdünnt wurde, versetzt und anschließend, wie in Beispiel 1 beschrieben, aufgearbeitet. Es wird ein weißes, geruchloses Pulver erhalten.

Beispiel 5

Es wird analog Beispiel 1 verfahren. Im Reaktionsgefäß werden 50 g eines Pentaerythritesters, in dem 3 OH-Gruppen mit Talgfettsäure (Gemisch aus geradkettigen Alkylmonocarbonsäuren folgender C-Kettenlängenverteilung: $C_{12}$=1 Gew.-%; $C_{14}$=3 Gew.-%; $C_{16}$=31 Gew.-%; $C_{18}$=65 Gew.-%) verestert sind (=0,0556 mol) in 200 cm³ Ethyldiisopropylamin gelöst und unter Rühren bei einer auf 25°C konstant gehaltenen Temperatur während 52 Minuten ein neben Stickstoff 9,1 Vol.-% SO₃ enthaltendes Gas über die Oberfläche der Lösung geleitet. Es werden 4,79 g SO₃ (=0,0598 mol) von der Lösung aufgenommen, das sind 1,07 mol SO₃ je 1 mol des Pentaerythritesters. Nach Beendigung der SO₃-Einleitung wird die Reaktionsmischung während 1 h unter Rühren auf 100°C erwärmt. Eine Probe des Reaktionsgemisches enthält nach Abdampfung des Lösungsmittels 88,9 Gew.-% des Ethyl-diisopropylamin-salzes vom sulfatierten Pentaerythrit-Ester (bestimmt nach Epton), es enthält ferner 3,4 Gew.-% Sulfationen und hat eine Säurezahl von 96 mg KOH/g. Das Reaktionsgemisch wird nun mit 8,8 g einer 30 gew.-%igen wäßrigen Natronlauge, die mit 10 cm³ Ethyl-diisopropylamin verdünnt wurde, versetzt und anschließend, wie in Beispiel 1 beschrieben, aufgearbeitet. Es wird eine beigefarbene, geruchlose, zähe, kristalline Masse erhalten.

Beispiel 6

Es wird analog Beispiel 1 verfahren. Im Reaktionsgefäß werden 50 g eines technischen Gemisches, welches nach geschromatographischer Analyse 17 Gew.-% Diglycerin, 34 Gew.-% Diglycerinmonolaurat, 28 Gew.-% Diglycerindilaurat, 11 Gew.-% Diglycerintrilaurat und 10 Gew.-% eines Restes, bestehend aus Glycerin, Glycerinmono-, -di- und -trilaurat, enthält, in 250 cm³ Tri-n-propylamin gelöst. Das eingesetzte, technische, esterhaltige Gemisch hat eine OH-Zahl von 432 mgKOH/g, aufgrund Kernresonanz (NMR-) spektroskopischer Analyse enthält das Gemisch 37,5% primäre OH-Gruppen und 62,5% sekundäre OH-Gruppen (Prozentzahlen jeweils bezogen auf Gesamtmenge der OH-Gruppen). Daraus folgt, daß von der gesamten OH-Zahl 162 mgKOH/g auf primäre OH-Gruppen und 270 mgKOH/g auf sekundäre OH-Gruppen zurückzuführen sind.

In die Lösung des Gemisches werden unter Rühren bei einer auf 25°C konstant gehaltenen Temperatur während 4,5 h ein neben Stickstoff 4,8 Vol.-% SO₃ enthaltendes Gas eingeleitet. Es werden 13,6 g SO₃ (=0,17 mol) von der Lösung aufgenommen, das sind je mol primäre OH-Gruppen 1,18 mol SO₃. Nach Beendigung der SO₃-Einleitung wird die Reaktionsmischung während 1 h unter Rühren weiter bei 25°C gehalten, anschließend wird das Reaktionsgemisch mit 24,8 g einer 30 gew.-%igen wäßrigen Natronlauge, die mit 50 cm³ Tri-n-propylamin verdünnt wurde, versetzt und wie in Beispiel 1 beschrieben, aufgearbeitet. Es wird eine helle, geruchlose, zähe, kristalline Masse erhalten, die 31 Gew.-% Natriumsalz des sulfatierten Diglycerinmonolaurats und 6,8 Gew.-% Natriumsulfat enthält.

**Patentansprüche**

1. Verfahren zur Sulfatierung primärer, freier OH-Gruppen von Teilestern aliphatischer, mehrwertiger Alkohole mit SO₃ in Gegenwart eines Lösungsmittels, dadurch gekennzeichnet, daß als Lösungsmittel mindestens eine Verbindung eingesetzt wird, die bei 98 kPa Druck einen Siedepunkt von 40 bis 200°C aufweist, unter den gewählten Reaktionsbedingungen flüssig ist und neben mindestens einem dreiwertigen N-Atom, das ausschließlich mit C-Atomen verbunden ist, nur C-, H- und gegebenenfalls Ethersauerstoff-Atome enthält.

2. Verfahren nach Anspruch 1, dadurch gekennzeichnet, daß je 1 mol primäre, freie OH-Gruppe des Teilesters 0,9 bis 1,2 mol SO₃ verwendet werden.

3. Verfahren nach Anspruch 1 oder 2, dadurch gekennzeichnet, daß die Umsetzung des Teilesters mit dem SO₃ bei 0 bis 60°C erfolgt.

4. Verfahren nach einem oder mehreren der Ansprüche 1 bis 3, dadurch gekennzeichnet, daß das SO₃ gasförmig in Mischung mit einem Inertgas eingesetzt wird, wobei diese Mischung 1 bis 30 Vol.-% SO₃ enthält.

5. Verfahren nach einem oder mehreren der Ansprüche 1 bis 4, dadurch gekennzeichnet, daß das Reaktionsgemisch nach Beendigung der SO₃-Zugabe und gegebenenfalls einer Nachreaktionszeit mit Alkali- oder Erdalkalihydroxid versetzt und anschließend das Lösungsmittel abdestilliert wird.

6. Verfahren nach Ansprüchen 2 und 5, dadurch gekennzeichnet, daß je 1 mol eingesetztes SO₃ bis 1,4 Äquivalente des Alkali- oder Erdalkalihydroxids verwendet werden.

7. Verfahren nach einem oder mehreren der Ansprüche 1 bis 6, dadurch gekennzeichnet, daß als Teilester mindestens ein Carbonsäuremonoester des Glycerins, Diglycerins, Ethylenglykols oder Diethylenglykols eingesetzt wird.

8. Verfahren nach einem oder mehreren der Ansprüche 1 bis 7, dadurch gekennzeichnet, daß als Lösungsmittel mindestens eine Verbindung eingesetzt wird, die bei 98 kPa Druck einen Siedepunkt von 60 bis 150°C aufweist.

9. Verfahren nach einem oder mehreren der Ansprüche 1 bis 8, dadurch gekennzeichnet, daß als Lösungsmittel mindestens eine Verbindung eingesetzt wird, die ein dreiwertiges Stickstoffatom enthält, das mit drei Kohlenstoffatomen verbunden ist.

**Revendications**

1. Procédé pour sulfater des radicaux OH primaires libres contenus dans des esters partiels de polyols aliphatiques, avec SO₃ en présence d'un solvant, procédé caractérisé en ce qu'on utilise, comme solvant, au moins un composé qui a un point d'ébulition de 40 à 200°C sous une pression de 98 kPa, qui est liquide dans les conditions réactionnelles appliquées et qui, en plus d'au moins d'un atome d'azote trivalent lié exclusivement à des atomes de carbone, ne contient que des atomes de carbone et d'hydrogène et, éventuellement, des atomes d'oxygène de fonction éther.

2. Procédé selon la revendication 1 caractérisé en ce qu'on utilise, par mole de radical OH primaire libre de l'ester partiel, de 0,9 à 1,2 mol de SO₃.

3. Procédé selon l'une des revendications 1 et 2, caractérisé en ce que la réaction de l'ester partiel avec SO₃ est exécutée à une température de 0 à 60°C.

4. Procédé selon une ou plusiers des revendications 1 à 3, caractérisé en ce que le SO₃ est mis en jeu à l'état gazeaux sous la forme d'un mélange avec un gaz inerte contenant de 1 à 30% en volume de SO₃.

5. Procédé selon une ou plusieurs des revendications 1 à 4, caractérisé en ce qu'on ajoute au mélange réactionnel, après la fin de l'addition de SO₃ et, éventuellement après un temps de réaction complémentaire, un hydroxyde de métal alcalin ou de métal alcalinoterreux, puis on chasse le solvant par distillation.

6. Procédé selon les revendications 2 et 5, caractérisé en ce qu'on utilise par mole du SO₃ mit en jeu de 1 à 1,4 équivalent de l'hydroxyde de métal alcalin ou de métal alcalinoterreux.

7. Procédé selon une ou plusieurs des revendications 1 à 6, caractérisé en ce qu'on utilise, comme ester partiel, au moins un mono-ester d'acide carboxylique du glycérol, du diglycérol, de l'éthylèneglycol ou du di-éthylène-glycol.

8. Procédé selon une ou plusieurs des revendications 1 à 7, caractérisé en ce qu'on utilise, comme solvant, au moins un composé ayant une temperature d'ébullition de 60 à 150°C sous une pression de 98 kPa.

9. Procédé selon une ou plusieurs des revendications 1 à 8, caractérisé en ce qu'on utilise, comme solvant, au moins un composé qui contient un atome s'azote trivalent lié à trois atomes de carbone.

**Claims**

1. A process for the sulfation with SO₃ of primary free OH groups of partial esters of aliphatic polyhydric alcohols in the presence of a solvent, characterised in that a compound is used as solvent which has a boiling point of 40 to 200°C at a pressure of 98 kPa, is liquid under the chosen reaction conditions and in addition to at least one trivalent nitrogen atom, which is linked exclusively to carbon atoms, contains only carbon, hydrogen and optionally ether oxygen atoms.

2. The process as claimed in claim 1, characterised in that 0.9 to 1.2 mol of SO₃ are used per 1 mol of primary free OH groups of the partial ester.

3. The process as claimed in claim 1 or 2, characterised in that the reaction of the partial ester with the SO₃ is carried out at 0 to 60°C.

4. The process as claimed in one or more of the claims 1 to 3, characterised in that the SO₃ is used in gaseous form mixed with an inert gas, this mixture containing 1 to 30% by volume of SO₃.

5. The process as claimed in one or more of the claims 1 to 4, characterised in that, after stopping the addition of SO₃ and optionally after a post reaction time, an alkali hydroxide or alkaline earth hydroxide is added to the reaction mixture and the solvent is then distilled off.

6. The process as claimed in claims 2 and 5, characterised in that 1 to 1.4 equivalents of the alkali hydroxide or alkaline earth hydroxide are used per 1 mol of SO₃ employed.

7. The process as claimed in one or more of the claims 1 to 6, characterised in that at least one carboxylic acid monoester of glycerol, diglycerol, ethylene glycol or diethylene glycol is used as partial ester.

8. The process as claimed in one or more of the claims 1 to 7, characterised in that a compound is used as solvent which has a boiling point of 60 to 150°C at a pressure of 98 kPa.

9. The process as claimed in one or more of the claims 1 to 8, characterised in that a compound is used as solvent which contains a trivalent nitrogen atom which is linked to three carbon atoms.